# EUROPEAN PATENT APPLICATION

(11) **EP 4 675 301 A1**
(43) Date of publication of application: **07.01.2026**
(21) Application number: 25186055.7
(22) Date of filing: 27.06.2025
(51) Int. Cl.: G01R 33/3415, G01R 33/34

(54) **RF COIL ARRAY**

(30) Priority: 01.07.2024 JP 2024106307
(71) Applicant: FUJIFILM Corporation, Tokyo Tokyo 106-8620 (JP)
(72) Inventor: OTAKE, Yosuke, Tokyo, 1068620 (JP); IWASAWA, Kojiro, Tokyo, 1068620 (JP)
(74) Representative: Dehns Germany Partnerschaft mbB

(57) **Abstract**

An RF coil array (100) includes: a first coil unit (102) including a first fixing belt (104) formed of a first stretch material stretchable in a first direction and RF coils independently disposed on the first fixing belt; and a second coil unit (122) including a second fixing belt (124) formed of a second stretch material stretchable in the first direction and RF coils independently disposed on the second fixing belt. The first coil unit is mounted on a subject with a distal end of the first fixing belt directed from one side to the other side of the subject in the first direction, and the second coil unit is mounted on the subject with a distal end of the second fixing belt directed from the other side to the one side of the subject in the first direction such that at last a part of the second coil unit overlaps the first coil unit.

## Description

### BACKGROUND OF THE INVENTION

### 1. Field of the Invention

The present invention relates to an RF coil array, and particularly to a technology of fixing an RF coil array for a magnetic resonance imaging apparatus to a subject.

### 2. Description of the Related Art

A magnetic resonance imaging apparatus (hereinafter referred to as an MRI apparatus) receives a magnetic resonance signal generated in a subject, and reconstructs the received signal to obtain a magnetic resonance image. In such an MRI apparatus, it is necessary to fix an RF coil array in which a plurality of radio frequency (RF) coils for receiving a magnetic resonance signal are disposed to a subject.

US10209326A describes an MR coil including at least one antenna element, in which the antenna element has at least one expandable conductor section comprising a core that includes a plastic cover and a conductive fluid.

### SUMMARY OF THE INVENTION

By disposing an RF coil near the subject, relatively high sensitivity can be obtained. However, there are cases where a one-piece RF coil array with a fixed size cannot accommodate variations in size of the subject. In such cases, for a large subject, the RF coil cannot be disposed to cover the body of the subject, resulting in a problem that the sensitivity of the RF coil decreases and a sufficient signal-to-noise ratio (SNR) of the magnetic resonance signal cannot be obtained.

Contrary to this, the MR coil described in US10209326A expands and contracts to be in close contact with the body of the subject even in a case where the size of the subject changes, and provides an appropriate SNR. However, the MR coil described in US10209326A uses a special electrical component, which poses a problem of cost.

The present invention has been made in view of such circumstances, and an object of the present invention is to provide an RF coil array capable of obtaining a relatively high sensitivity for subjects having various sizes by solving at least one of the above-described problems.

In order to achieve the above object, according to a first aspect of the present disclosure, there is provided an RF coil array for a magnetic resonance imaging apparatus, comprising: a first coil unit including a first fixing belt formed of a first stretch material that is stretchable in a first direction and a plurality of RF coils each independently disposed on the first fixing belt; and a second coil unit including a second fixing belt formed of a second stretch material that is stretchable in the first direction and a plurality of RF coils each independently disposed on the second fixing belt, in which each of the plurality of RF coils of the first coil unit has a center disposed on a first straight line, and the RF coils closer to a distal end of the first fixing belt have smaller diameters, each of the plurality of RF coils of the second coil unit has a center disposed on a second straight line, and the RF coils closer to a distal end of the second fixing belt have smaller diameters, the first straight line and the second straight line are disposed offset from each other in a second direction intersecting the first direction, the first coil unit is mounted on a subject with the distal end of the first fixing belt directed from one side to the other side of the subject in the first direction, and the second coil unit is mounted on the subject with the distal end of the second fixing belt directed from the other side to the one side of the subject in the first direction such that at least a part of the second coil unit overlaps the first coil unit.

It is preferable that a second aspect of the present disclosure relates to the RF coil array according to the first aspect, in which the first straight line and the second straight line are each parallel to the first direction.

It is preferable that a third aspect of the present disclosure relates to the RF coil array according to the first or second aspect, in which, in a case where the first coil unit and the second coil unit are mounted on the subject, any of the plurality of RF coils of the first coil unit and any of the plurality of RF coils of the second coil unit at least partially overlap each other.

It is preferable that a fourth aspect of the present disclosure relates to the RF coil array according to the first or second aspect, in which, in a case where the first coil unit and the second coil unit are mounted on the subject, the RF coil having a largest diameter among the plurality of RF coils of the first coil unit and the RF coil having a smallest diameter among the plurality of RF coils of the second coil unit at least partially overlap each other.

It is preferable that a fifth aspect of the present disclosure relates to the RF coil array according to any one of the first to fourth aspects, in which a part of at least one of the first fixing belt or the second fixing belt is formed of a non-stretch material having a smaller elongation ratio in the first direction than the first stretch material and the second stretch material.

It is preferable that a sixth aspect of the present disclosure relates to the RF coil array according to any one of the first to fifth aspects further comprising: a stopper structure that restricts elongation of the first fixing belt and the second fixing belt in the first direction beyond a predetermined amount.

It is preferable that a seventh aspect of the present disclosure relates to the RF coil array according to the sixth aspect, in which the stopper structure includes a stopper band that is formed of a non-stretch material having a smaller elongation ratio in the first direction than the first stretch material and the second stretch material and that is provided on the first fixing belt and the second fixing belt with a bend provided in advance in the first direction.

It is preferable that an eighth aspect of the present disclosure relates to the RF coil array according to any one of the first to seventh aspects, in which, in the first coil unit and the second coil unit, an end part of each of the first fixing belt and the second fixing belt on an opposite side to the distal end is connected to a top plate on which the subject is placed.

It is preferable that a ninth aspect of the present disclosure relates to the RF coil array according to the eighth aspect, in which the first fixing belt and the second fixing belt each comprises a rail hook that is engaged with a rail groove provided in the top plate and that is movable along the rail groove at the end part on the opposite side.

It is preferable that a tenth aspect of the present disclosure relates to the RF coil array according to the eighth or ninth aspect further comprising: a tightening mechanism for adjusting an elongation amount of at least one of the first fixing belt or the second fixing belt in the first direction after being mounted on the subject.

It is preferable that an eleventh aspect of the present disclosure relates to the RF coil array according to the tenth aspect, in which the tightening mechanism comprises an annular member connected to the top plate, and the end part of at least one of the first fixing belt or the second fixing belt on the opposite side to the distal end is passed through an inside of the annular member and folded back to be fixed.

It is preferable that a twelfth aspect of the present disclosure relates to the RF coil array according to any one of the first to seventh aspects, in which the first coil unit and the second coil unit are each connected to a connection unit to be integrated.

It is preferable that a thirteenth aspect of the present disclosure relates to the RF coil array according to the twelfth aspect, in which the connection unit includes a plurality of RF coils.

It is preferable that a fourteenth aspect of the present disclosure relates to the RF coil array according to the twelfth or thirteenth aspect further comprising: a tightening mechanism for adjusting an elongation amount of at least one of the first fixing belt or the second fixing belt in the first direction after being mounted on the subject.

It is preferable that a fifteenth aspect of the present disclosure relates to the RF coil array according to the fourteenth aspect, in which the tightening mechanism comprises a tightening belt connected to at least one of the first fixing belt or the second fixing belt, and an annular member, and the tightening belt is passed through an inside of the annular member and folded back to be fixed.

It is preferable that a sixteenth aspect of the present disclosure relates to the RF coil array according to any one of the first to fifteenth aspects, in which each of the plurality of RF coils of the first coil unit overlaps an adjacent RF coil, and each of the plurality of RF coils of the second coil unit overlaps an adjacent RF coil.

It is preferable that a seventeenth aspect of the present disclosure relates to the RF coil array according to any one of the first to sixteenth aspects, in which the first fixing belt and the second fixing belt comprise first surfaces and second surfaces of a pair of hook-and-loop fasteners each having the first surface and the second surface engaging with each other, at positions where the first surface and the second surface come into contact with each other in a case where the first coil unit and the second coil unit overlap the subject and are mounted on the subject.

It is preferable that an eighteenth aspect of the present disclosure relates to the RF coil array according to any one of the first to seventeenth aspects, in which the first coil unit is mounted on the subject with the first fixing belt elongated, and the second coil unit is mounted on the subject with the second fixing belt elongated.

According to the present invention, it is possible to obtain a relatively high sensitivity for subjects of various sizes.

### BRIEF DESCRIPTION OF THE DRAWINGS

Fig. 1 is a perspective view of an example of an MRI apparatus.
Fig. 2 is a sectional schematic view taken along line 2-2 of Fig. 1.
Fig. 3 is a perspective view showing an example of the MRI apparatus according to the present disclosure.
Figs. 4A and 4B are plan views of a first coil unit and a second coil unit.
Fig. 5 is a perspective view showing an example of the MRI apparatus.
Fig. 6 is a cross-sectional view taken along line 6-6 of Fig. 5.
Figs. 7A and 7B are top perspective views of the first coil unit and the second coil unit.
Figs. 8A and 8B are views for describing a relationship between a size of a subject and disposition of RF coils.
Figs. 9A and 9B are views for describing a relationship between a size of a subject and disposition of the RF coils.
Figs. 10A and 10B are views for describing a relationship between a size of a subject and disposition of the RF coils.
Figs. 11A and 10B are top perspective views of the first coil unit.
Figs. 12A and 12B are top perspective views of the first coil unit.
Figs. 13A and 13B are views showing an RF coil array.
Figs. 14A and 14B are views showing an RF coil array.
Figs. 15A and 15B are views showing an RF coil array.
Fig. 16 is a view showing an RF coil array.
Fig. 17 is a view showing an RF coil array.

### DESCRIPTION OF THE PREFERRED EMBODIMENTS

Hereinafter, preferred embodiments of an RF coil array according to the present disclosure will be described with reference to the accompanying drawings. In the present specification, the same components are denoted by the same reference numerals, and duplicate description thereof will be omitted as appropriate.

### Configuration of Typical Apparatus

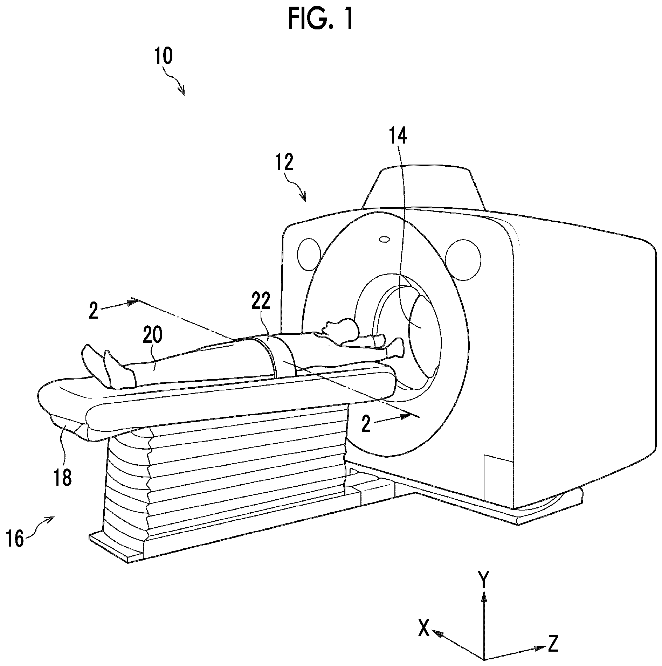

Fig. 1 is a perspective view showing an example of a typical MRI apparatus 10. As shown in Fig. 1, the MRI apparatus 10 comprises a magnet 12 that generates a static magnetic field and a bed 16. The magnet 12 has a cylindrical examination (imaging) space 14. In addition, the magnet 12 is provided with an MRI magnet (not shown) as a magnetic field generating source, and various other coils (not shown).

The bed 16 is installed on a front surface side of the magnet 12 to face the examination space 14. The bed 16 comprises a top plate 18. A subject 20 is placed on the top plate 18 along a longitudinal direction of the top plate 18. The top plate 18 is configured to be movable in an X direction that is a width direction perpendicular to the longitudinal direction of the top plate 18, a Y direction that is a vertical direction, and a Z direction that is the longitudinal direction of the top plate 18.

The MRI apparatus 10 comprises a drive mechanism (not shown) that moves the top plate 18 in the Z direction to enter the examination space 14 and exit the examination space 14. In the MRI apparatus 10, the top plate 18 is moved in the examination space 14, so that an examination target region of the subject 20 to be imaged, placed on the top plate 18, is set to a center of the static magnetic field in the examination space 14.

A multi-channel RF coil array (not shown) consisting of a plurality of RF coils for receiving the magnetic resonance signal generated in the subject 20 is fixed to the examination target region of the subject 20 by a fixing belt 22. In the example shown in Fig. 1, an abdomen of the subject 20 is the examination target region, and the RF coil array is mounted on the abdomen of the subject 20.

A reception-side cable (not shown) that outputs the magnetic resonance signal received by the RF coil is connected to the RF coil array. A reception-side connector (not shown) is connected to an end part of the reception-side cable. The reception-side connector is connected to a bed-side connector of a bed-side cable (not shown). Accordingly, the reception-side cable and the bed-side cable are communicatively connected to each other via the connectors.

The bed-side cable is stored in a cable storage portion (not shown) of the bed 16. The magnetic resonance signal of the subject 20 received by the RF coil is transmitted to a signal processing unit (not shown) via the reception-side cable and the bed-side cable. The signal processing unit performs signal processing on the received magnetic resonance signal to convert the received magnetic resonance signal into an image signal.

### Problem 1

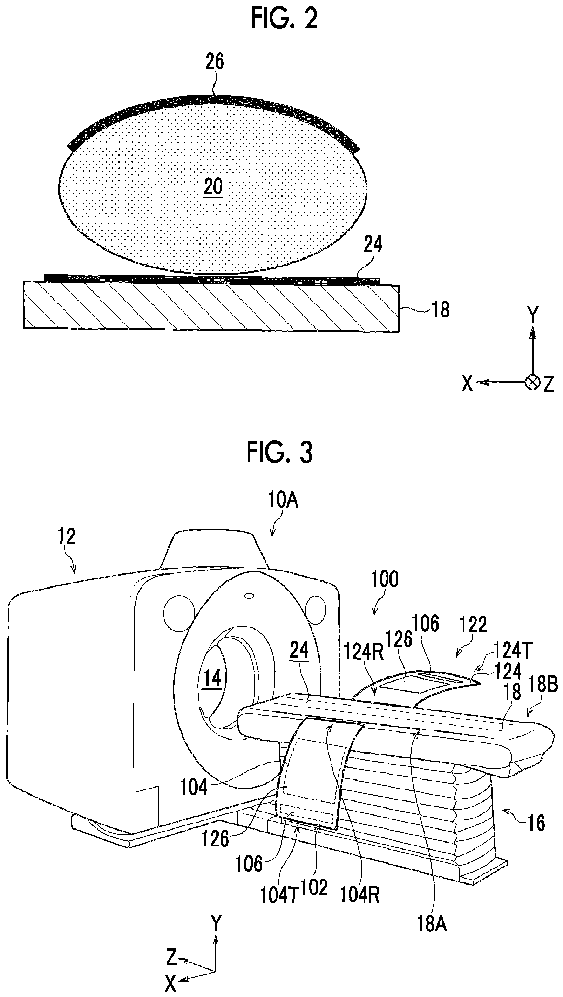

Fig. 2 is a schematic sectional view taken along line 2-2 of Fig. 1. As shown in Fig. 2, a back (spine) coil 24 comprising a plurality of RF coils (not shown) is disposed on a surface of the top plate 18 on which the subject 20 is placed. Further, an upper abdomen coil 26 fixed by the fixing belt 22 (not shown in Fig. 2) is disposed on the abdomen of the subject 20 placed on the top plate 18. The back coil 24 and the upper abdomen coil 26 correspond to the RF coil array.

It is preferable that the RF coils of the RF coil array are disposed near the subject 20 with no gaps. However, in the case of a one-piece RF coil array having a fixed size, such as the upper abdomen coil 26, the variation in the size of the subject 20 cannot be covered, and in a range that cannot be covered by the RF coils, a sufficient SNR may not be obtained. In the example shown in Fig. 2, there is a region on a side surface of the subject 20 (left and right surfaces of the subject 20 in Fig. 2) that is not covered by any one of the back coil 24 and the upper abdomen coil 26.

### Problem 2

On the other hand, in the MR coil described in US10209326A, an expandable conductor is configured by using a plastic cover filled with a high-quality conductive fluid, so that length adjustment is possible, and the MR coil can be brought into close contact even in a case where the size of the subject is changed. However, there is a problem in that the cost is higher than in a conductive material in the related art.

### Configuration of Apparatus of Present Disclosure

Fig. 3 is a perspective view showing an example of an MRI apparatus 10A according to embodiment of the present disclosure. As shown in Fig. 3, the MRI apparatus 10A comprises an RF coil array 100 for an MRI apparatus. The RF coil array 100 comprises a first coil unit 102 and a second coil unit 122.

The first coil unit 102 comprises a first fixing belt 104. The first fixing belt 104 has a proximal end 104R disposed on a first end 18A side (a front side of the top plate 18 in Fig. 3) of the top plate 18 in the X direction, and a distal end 104T that is a free end.

The second coil unit 122 comprises a second fixing belt 124. The second fixing belt 124 has a proximal end 124R disposed on a second end 18B side (an inner side of the top plate 18 in Fig. 3) opposite to the first end 18A side of the top plate 18 in the X direction, and a distal end 124T that is a free end.

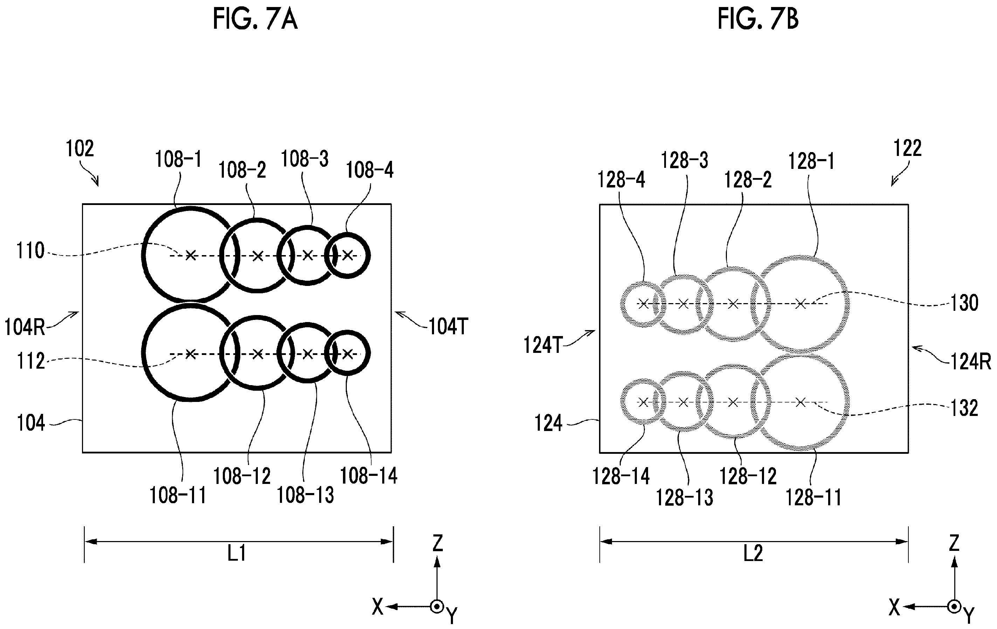

The first fixing belt 104 is formed of a first stretch material that is stretchable in an elongation direction (an example of a "first direction", the X direction in a state shown in Figs. 7A and 7B) in which the proximal end 104R and the distal end 104T are separated from each other. The second fixing belt 124 is formed of a second stretch material that is stretchable in an elongation direction in which the proximal end 124R and the distal end 124T are separated from each other.

The stretch material refers to either a polyurethane-based elastic fiber that is inherently extensible, maintaining a non-elongated state under no-load conditions where no tensile force is applied, and deforming into an elongated state by elongating, when a tensile force is generated in an elongation direction, in the elongation direction in response to the tensile force, while also attempting to return to its original non-elongated state due to its restoring force; or a polyester-based fiber material in which a mechanical structure is provided through the method of fiber weaving. In a case where the tensile force is released in the elongated state and the stretch material is returned to a no-load condition, the stretch material restores to the non-elongated state due to a restoring force thereof. An elongation ratio [%] of the stretch material in the elongation direction is preferably 20% or more, and more preferably 30% or more. In the present specification, the elongation ratio is a value measured in accordance with JIS L 1096 Method B.

In addition, the stretch material is preferably a one-way material (an asymmetric material that does not extend in a non-elongation direction orthogonal to the elongation direction) that is likely to elongate only in one direction. It is preferable that the stretch material has a structure in which at least the elongation ratio in the non-elongation direction is smaller than the elongation ratio in the elongation direction. The one-way material may have a structure in which, for example, a material having no elasticity is partially disposed in a non-elongation direction in a band shape on a stretch material.

It is preferable that the elongation ratio of the first stretch material in the elongation direction and the elongation ratio of the second stretch material in the elongation direction are equal to each other. The first stretch material and the second stretch material may be the same material.

The proximal end 104R of the first fixing belt 104 and the proximal end 124R of the second fixing belt 124 are disposed at the same coordinate position in the Z direction of the top plate 18 in the Z direction. The first fixing belt 104 and the second fixing belt 124 are configured to have the same length in the Z direction. In addition, the first fixing belt 104 and the second fixing belt 124 are configured such that a length from the proximal end 104R to the distal end 104T and a length from the proximal end 124R to the distal end 124T are the same. That is, the first fixing belt 104 and the second fixing belt 124 have the same shape and are disposed in left-right symmetry with respect to the top plate 18.

The first fixing belt 104 and the second fixing belt 124 are each provided with a first surface (A surface) 106 and a second surface (B surface) 126 of a hook-and-loop fastener at positions that overlap each other in a case where the RF coil array 100 is mounted on the subject 20 (not shown in Fig. 3). The hook-and-loop fastener is a coupling member in which a hook surface and a loop surface forming a pair engage with each other. For example, the first surface 106 is a hook surface, and the second surface 126 is a loop surface.

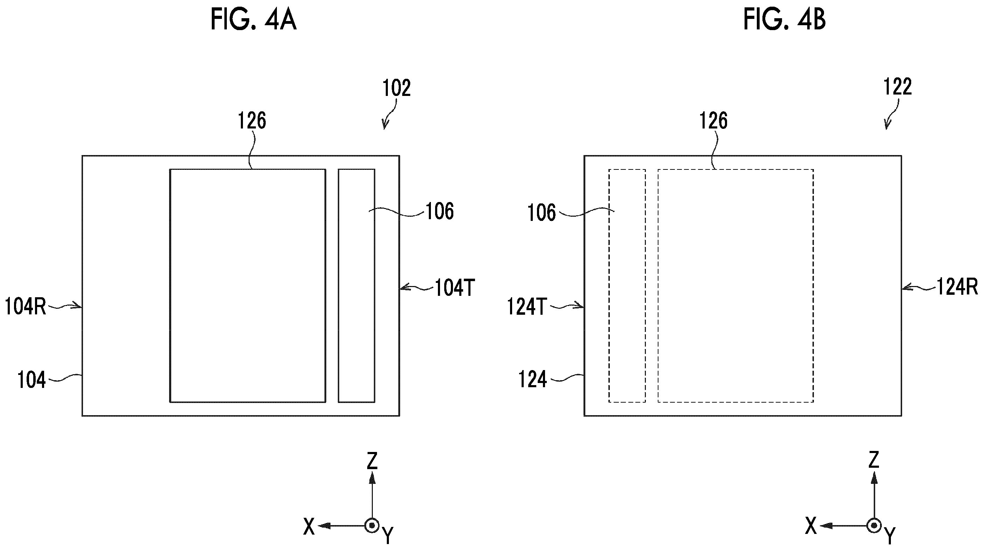

Figs. 4A and 4B are plan views of the first coil unit 102 and the second coil unit 122. Fig. 4A shows a state in which the first coil unit 102 is placed flat on the top plate 18 (not shown in Figs. 4A and 4B). Fig. 4B shows a state in which the second coil unit 122 is placed flat on the top plate 18. As shown in Fig. 4A, the first surface 106 of the hook-and-loop fastener is disposed in a band shape in a direction orthogonal to the elongation direction on the distal end 104T of a surface of the first fixing belt 104 that comes into contact with the second fixing belt 124. Further, the second surface 126 of the hook-and-loop fastener is disposed on the same surface.

In addition, as shown in Fig. 4B, the second surface 126 of the hook-and-loop fastener is disposed in a band shape in a direction orthogonal to the elongation direction on the distal end 124T of a surface of the second fixing belt 124 that comes into contact with the first fixing belt 104. Further, the second surface 126 of the hook-and-loop fastener is disposed on the same surface.

At least the second surface 126 of the hook-and-loop fastener is formed of a stretch material. In a case where a tensile force is generated in the elongation direction of the first fixing belt 104 and the second fixing belt 124, the second surface 126 of the hook-and-loop fastener elongates together with the elongation of the first fixing belt 104 and the second fixing belt 124 in response to the tensile force.

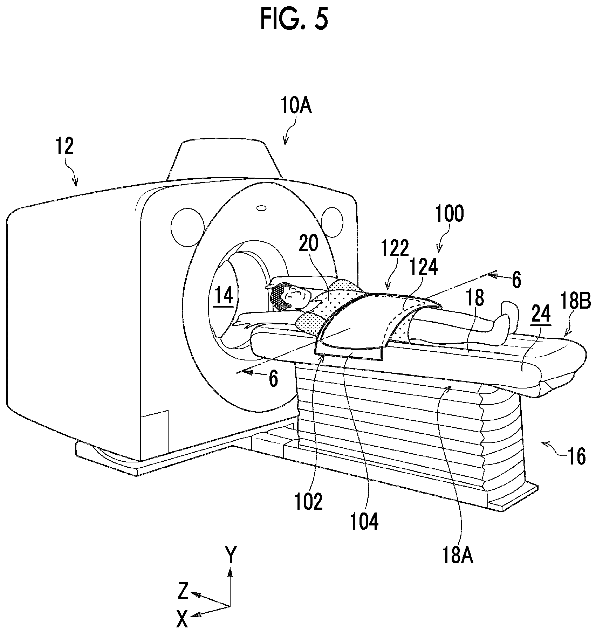

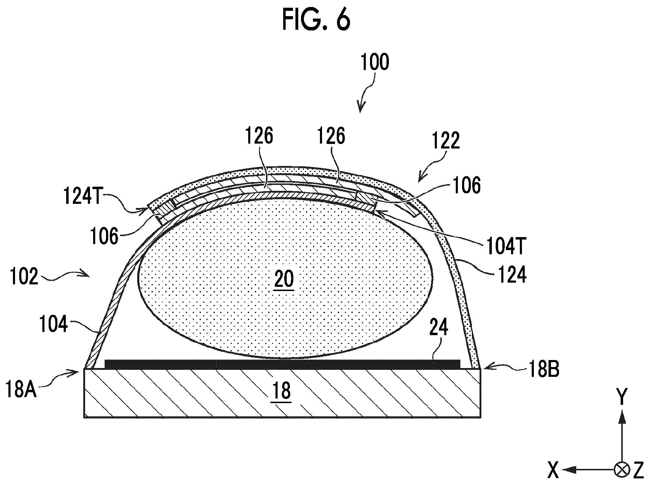

Fig. 5 is a perspective view showing an example of the MRI apparatus 10A and shows a state in which the RF coil array 100 is mounted on the subject 20 placed on the top plate 18. Further, Fig. 6 is a sectional view taken along line 6-4 of Fig. 5.

In order to mount the RF coil array 100 on the subject 20, a user first places the subject 20 on the top plate 18 and places the first coil unit 102 on the examination target region (here, the abdomen) of the subject 20. That is, the first coil unit 102 is mounted on the subject 20 with the distal end 104T of the first fixing belt 104 directed from the first end 18A side (an example of "one side in the first direction") to the second end 18B side (an example of "the other side in the first direction") in the X direction of the top plate 18.

Next, the user overlaps the second coil unit 122 with the first coil unit 102 mounted on the subject 20. In this case, the user couples the first surface 106 of the hook-and-loop fastener of the first fixing belt 104 with the second surface 126 of the hook-and-loop fastener of the second fixing belt 124, and the first surface 106 of the hook-and-loop fastener of the second fixing belt 124 with the second surface 126 of the hook-and-loop fastener of the first fixing belt 104. That is, the second coil unit 122 is mounted on the subject 20 with at least a part of the second coil unit 122 overlapping the first coil unit 102, with the distal end 124T of the second fixing belt 124 directed from the second end 18B side to the first end 18A side of the top plate 18.

In this way, the subject 20 is fixed to the top plate 18 by mounting the RF coil array 100 on the subject 20. Alternatively, the first surface 106 and the second surface 126 of the hook-and-loop fastener may be disposed such that the second coil unit 122 is first mounted on the subject 20 and the first coil unit 102 is mounted to overlap the second coil unit 122.

Figs. 7A and 7B are top perspective views of the first coil unit 102 and the second coil unit 122. Fig. 7A shows a state in which the first coil unit 102 is placed flat on the top plate 18 (not shown in Figs. 7A and 7B). In Fig. 7A, the first fixing belt 104 is in a non-elongated state in which no tensile force is applied in the X direction. A length of the first fixing belt 104 in the X direction in the non-elongated state is L1.

As shown in Fig. 7A, the first coil unit 102 includes a plurality of RF coils 108-1, 108-2, 108-3, 108-4, 108-11, 108-12, 108-13, and 108-14 that are two-dimensionally disposed on the first fixing belt 104. The RF coils 108-1 to 108-4 and 108-11 to 108-14 each have a circular shape in a plan view (when viewed in the Y direction in Figs. 7A and 7B).

The RF coils 108-1 to 108-4 and 108-11 to 108-14 are disposed on independent flexible substrates (not shown), respectively. Each flexible substrate is independently fixed to the first fixing belt 104 at a fixed point at a position of a center (indicated by a cross mark in Figs. 7A and 7B) of each of the RF coils 108-1 to 108-4 and 108-11 to 108-14.

In a plan view of the first fixing belt 104, the RF coils 108-1 to 108-4 partially overlap one another, and each have a center disposed on a straight line 110 (an example of a "first straight line"). For example, the RF coil 108-1 and the RF coil 108-2 partially overlap each other, the RF coil 108-2 and the RF coil 108-3 partially overlap each other, and the RF coil 108-3 and the RF coil 108-4 partially overlap each other.

Here, the straight line 110 is parallel to the X direction which is the elongation direction of the first fixing belt 104. In the present specification, "parallel" may be completely parallel or substantially parallel. The term "substantially parallel" refers to a state in which the direction of the straight line 110 has an angle with respect to the X direction to such an extent that the purpose of the present disclosure of obtaining a relatively high sensitivity even for a subject having a relatively large size can be achieved.

In addition, diameters of the RF coils 108-1 to 108-4 become smaller toward the distal end 104T of the first fixing belt 104. That is, the RF coils 108-1, 108-2, 108-3, and 108-4 have diameters that decrease in this order.

Similarly, in a plan view of the first fixing belt 104, the RF coils 108-11 to 108-14 partially overlap one another, and each have a center disposed on a straight line 112 parallel to the X direction. In addition, diameters of the RF coils 108-11 to 108-14 become smaller toward the distal end 104T of the first fixing belt 104.

Fig. 7B shows a state in which the second coil unit 122 is placed flat on the top plate 18. In Fig. 7B, the second fixing belt 124 is in a non-elongated state in which no tensile force is applied in the X direction. A length of the second fixing belt 124 in the X direction in the non-elongated state is L2. It is preferable that L2 is equal to L1.

As shown in Fig. 7B, the second coil unit 122 includes a plurality of RF coils 128-1, 128-2, 128-3, 128-4, 128-11, 128-12, 128-13, and 128-14 that are two-dimensionally disposed on the second fixing belt 124. The RF coils 128-1 to 128-4 and 128-11 to 128-14 each have a circular shape in a plan view.

The RF coils 128-1 to 128-4 and 128-11 to 128-14 are disposed on independent flexible substrates (not shown), respectively. Each flexible substrate is independently fixed to the second fixing belt 124 at a fixed point at a position of a center of each of the RF coils 128-1 to 128-4 and 128-11 to 128-14.

In a plan view of the second fixing belt 124, the RF coils 128-1 to 128-4 partially overlap one another, and each have a center disposed on a straight line 130 (an example of a "second straight line") parallel to the X direction which is the elongation direction of the second fixing belt 124. Here, the RF coil 128-1 and the RF coil 128-2 partially overlap each other, the RF coil 128-2 and the RF coil 128-3 partially overlap each other, and the RF coil 128-3 and the RF coil 128-4 partially overlap each other.

In addition, diameters of the RF coils 128-1 to 128-4 become smaller toward to the distal end 124T of the second fixing belt 124. That is, the RF coils 128-1, 128-2, 128-3, and 128-4 are diameters that decrease in this order.

Similarly, in a plan view of the second fixing belt 124, the RF coils 128-11 to 128-14 partially overlap one another, and each have a center disposed on a straight line 132 parallel to the X direction. In addition, diameters of the RF coils 128-11 to 128-14 become smaller toward the distal end 124T of the second fixing belt 124.

In the following description, in a case where there is no need to distinguish between the RF coils 108-1 to 108-4 and 108-11 to 108-14, the RF coils 108-1 to 108-4 and 108-11 to 108-14 may be simply referred to as the RF coils 108. Similarly, in a case where there is no need to distinguish between the RF coils 128-1 to 128-4 and 128-11 to 128-14, the RF coils 128-1 to 128-4 and 128-11 to 128-14 may be simply referred to as the RF coils 128. The number and disposition of the RF coils 108 of the first coil unit 102 and the RF coils 128 of the second coil unit 122 are not limited to the example shown in Figs. 7A and 7B.

The straight lines 110 and 112 of the first coil unit 102 and the straight lines 130 and 132 of the second coil unit 122 are disposed offset from each other in the Z direction (an example of a "second direction") orthogonal to (an example of "intersecting") the elongation direction of the first fixing belt 104 and the second fixing belt 124. Here, "disposed offset from each other" means that the straight lines are not disposed in the same straight line. For example, the straight lines 110 and 112 of the first coil unit 102 and the straight lines 130 and 132 of the second coil unit 122 are alternately disposed along the Z direction. In the example shown in Figs. 7A and 7B, the straight line 110, the straight line 130, the straight line 112, and the straight line 132 are disposed in this order from top to bottom of Figs. 7A and 7B. As described above, the straight line 110 is not disposed in the same straight line as the straight line 130 and the straight line 132, and the straight line 112 is also not disposed in the same straight line as the straight line 130 and the straight line 132.

As described above, the RF coil 108 of the first coil unit 102 and the RF coil 128 of the second coil unit 122 are disposed in point symmetry with respect to a point on a straight line that bisects the top plate 18 (not shown in Figs. 7A and 7B, see Fig. 3) in the X direction.

The RF coil array 100 may output the magnetic resonance signals received by each of the RF coils 108 and each of the RF coils 128 through the cable or wireless communication via a signal processing circuit (not shown in Figs. 7A and 7B) including a signal amplifier.

### Size of Subject and Disposition of RF Coil

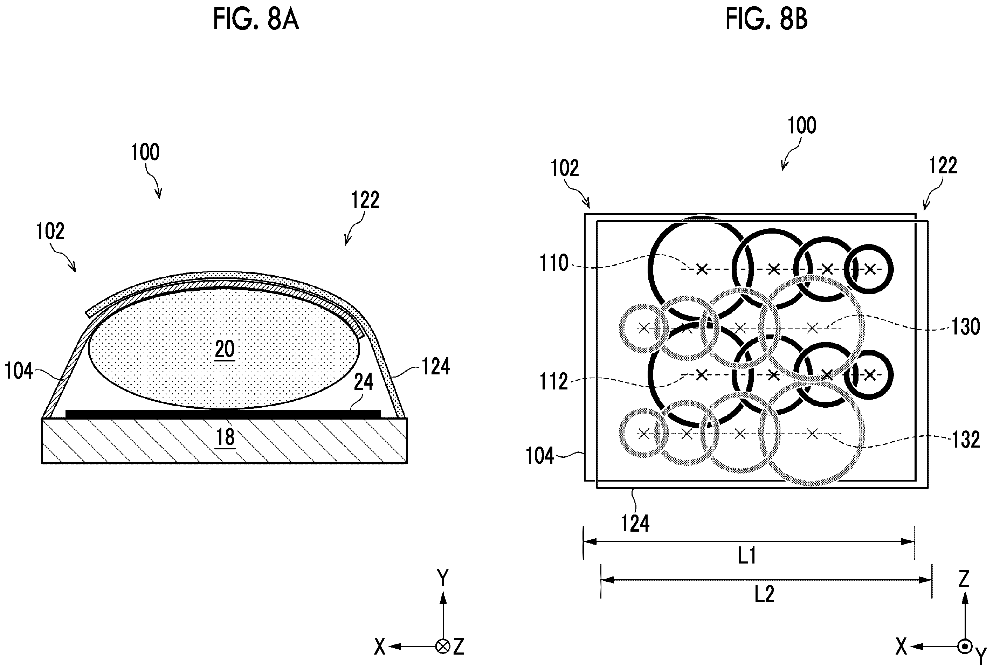

Figs. 8A to 10B are views for describing a relationship between a size of the subject and the disposition of the RF coils. Fig. 8A, Fig. 9A, and Fig. 10A are cross-sectional views similar to Fig. 6 in a state in which the RF coil array 100 is mounted on the subject 20. In addition, Fig. 8B, Fig. 9B, and Fig. 10B are top perspective views showing the disposition of the RF coils when the states of Fig. 8A, Fig. 9A, and Fig. 10A are viewed from a Z direction side, respectively, and are views showing the first fixing belt 104 and the second fixing belt 124 in a state of being deployed in the X direction. In Figs. 8A to 10B, the hook-and-loop fastener is not illustrated.

Figs. 8A and 8B show a state in which the RF coil array 100 is mounted on the subject 20 having a relatively small size (body type). In the state shown in Figs. 8A and 8B, the first fixing belt 104 and the second fixing belt 124 are both in a non-elongated state.

As shown in Fig. 8B, the RF coil array 100 covers a periphery of the examination target region of the subject 20 with no gaps using the RF coils 108-1 to 108-4, the RF coils 108-11 to 108-14, the RF coils 128-1 to 128-4, and the RF coils 128-11 to 128-14.

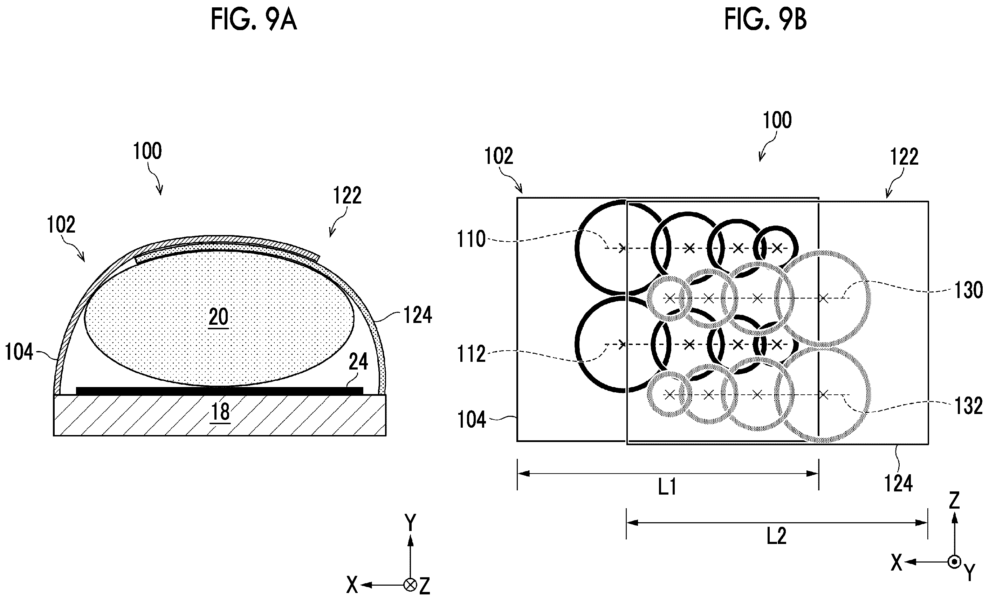

Figs. 9A and 9B show a state in which the RF coil array 100 is mounted on the subject 20 having a relatively medium size. In the state shown in Figs. 9A and 9B, the first fixing belt 104 and the second fixing belt 124 are both in a non-elongated state.

As shown in Figs. 8A to 9B, an overlapping length of the first fixing belt 104 and the second fixing belt 124 changes according to the size of the subject 20. That is, sizes of the first coil unit 102 and the second coil unit 122 in a peripheral direction of the subject 20 changes with gradations according to the size of the subject 20.

As shown in Fig. 9B, the RF coil array 100 covers the periphery of the examination target region of the subject 20 with no gaps using the RF coils 108-1 to 108-4, the RF coils 108-11 to 108-14, the RF coils 128-1 to 128-4, and the RF coils 128-11 to 128-14. As described above, even in a case where the overlapping length of the first fixing belt 104 and the second fixing belt 124 changes, the RF coils are disposed with a uniform density.

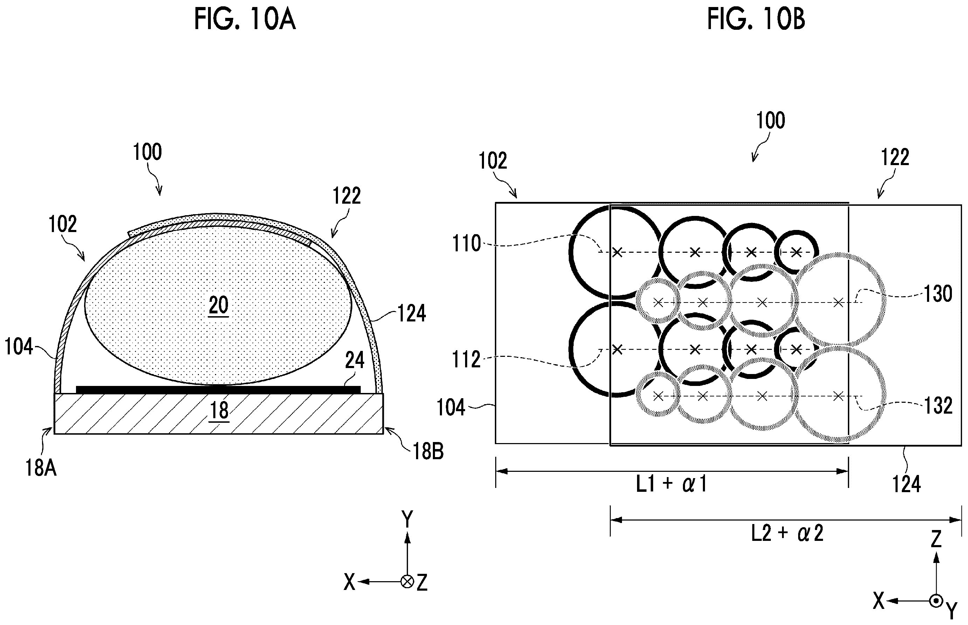

Figs. 10A and 10B show a state in which the RF coil array 100 is mounted on the subject 20 having a relatively large size. The first coil unit 102 is mounted on the subject 20 with the first fixing belt 104 elongated, and the second coil unit 122 is mounted on the subject 20 with the second fixing belt 124 elongated. In the state shown in Figs. 10A and 10B, the first fixing belt 104 and the second fixing belt 124 are in an elongated state of being elongated by α1 and α2, respectively.

In a case where the RF coil array 100 is mounted on the subject 20 having a relatively large size while the first fixing belt 104 and the second fixing belt 124 are both in the non-elongated state, the RF coil cannot be disposed to cover the examination target region. Contrary to this, by mounting the RF coil array 100 with the first fixing belt 104 and the second fixing belt 124 in the elongated state, the RF coils are disposed to cover the examination target region.

In order to mount the RF coil array 100 on the subject 20 having a relatively large size, the user further pulls the first coil unit 102 toward the second end 18B side of the top plate 18 from a state in which the first coil unit 102 is placed on the examination target region of the subject 20 placed on the top plate 18, and brings the first fixing belt 104 into an elongated state of being elongated by α1.

Next, the user pulls the second coil unit 122 toward the first end 18A side of the top plate 18 above the first coil unit 102 while maintaining the first fixing belt 104 in the elongated state, and brings the second fixing belt 124 into the elongated state of being elongated by α2. It is preferable that α2 is equal to α1. That is, it is preferable that the first fixing belt 104 and the second fixing belt 124 are evenly elongated.

Finally, the user overlaps the second coil unit 122 on the second fixing belt 124 in the elongated state with the first coil unit 102 with the first fixing belt 104 in the elongated state and couples the first surface 106 and the second surface 126 of the hook-and-loop fastener.

In the RF coil array 100 mounted in this way, distances between the centers of the plurality of RF coils 108-1, 108-2, 108-3, and 108-4 disposed on the straight line 110 of the first fixing belt 104 are larger than in a case where the first fixing belt 104 is in the non-elongated state. In addition, distances between the centers of the plurality of RF coils 108-11, 108-12, 108-13, and 108-14 disposed on the straight line 112 of the first fixing belt 104 are also relatively larger than in a case where the first fixing belt 104 is in the non-elongated state. The same applies to distances between the centers of the RF coils 128-1 to 128-4 and 128-11 to 128-14 disposed in the second fixing belt 124.

In this way, the first fixing belt 104 and the second fixing belt 124 are elongated, so that a length over which the RF coils are present in the X direction is widened, and the RF coils are disposed with a uniform density. As a result, even for the subject having a relatively large size that cannot be covered in a case where the first fixing belt 104 and the second fixing belt 124 are not elongated, a relatively high sensitivity can be obtained.

### Restriction on Disposition of RF Coil

It is preferable that, in a case where the first coil unit 102 and the second coil unit 122 are mounted on the subject 20, each of the plurality of RF coils 108 of the first coil unit 102 overlaps the adjacent RF coil 108 and each of the plurality of RF coils 128 of the second coil unit 122 overlaps the adjacent RF coil 128 even in a case where the first fixing belt 104 and the second fixing belt 124 are in the elongated state. In a case where a region where the adjacent RF coils do not overlap each other is formed, the sensitivity of the RF coils is relatively low.

It is preferable that, in a case where the first coil unit 102 and the second coil unit 122 are mounted on the subject 20, any of the plurality of RF coils 108 of the first coil unit 102 and any of the plurality of RF coils 128 of the second coil unit 122 at least partially overlap each other as viewed in a direction orthogonal to the surfaces of the first fixing belt 104 and the second fixing belt 124 (that is, a direction orthogonal to the peripheral direction of the subject 20). As a result, imaging of subjects of various sizes can be achieved.

In addition, It is preferable that, in a case where the first coil unit 102 and the second coil unit 122 are mounted on the subject 20, the RF coil 108 having the largest diameter (for example, the RF coil 108-1) among the plurality of RF coils 108 of the first coil unit 102 and the RF coil 128 having the smallest diameter (for example, the RF coil 128-4) among the plurality of RF coils 128 of the second coil unit 122 at least partially overlap each other as viewed in a direction orthogonal to the surfaces of the first fixing belt 104 and the second fixing belt 124. As a result, imaging of subjects of various sizes can be achieved.

Similarly, it is preferable that, in a case where the first coil unit 102 and the second coil unit 122 are mounted on the subject 20, the RF coil 108 having the smallest diameter (for example, the RF coil 108-4) among the plurality of RF coils 108 of the first coil unit 102 and the RF coil 128 having the largest diameter (for example, the RF coil 128-1) among the plurality of RF coils 128 of the second coil unit 122 at least partially overlap each other.

### Restriction on Elongation of RF Coil Array

A part of the first fixing belt may be formed of a non-stretch material having a small elongation ratio than the first stretch material.

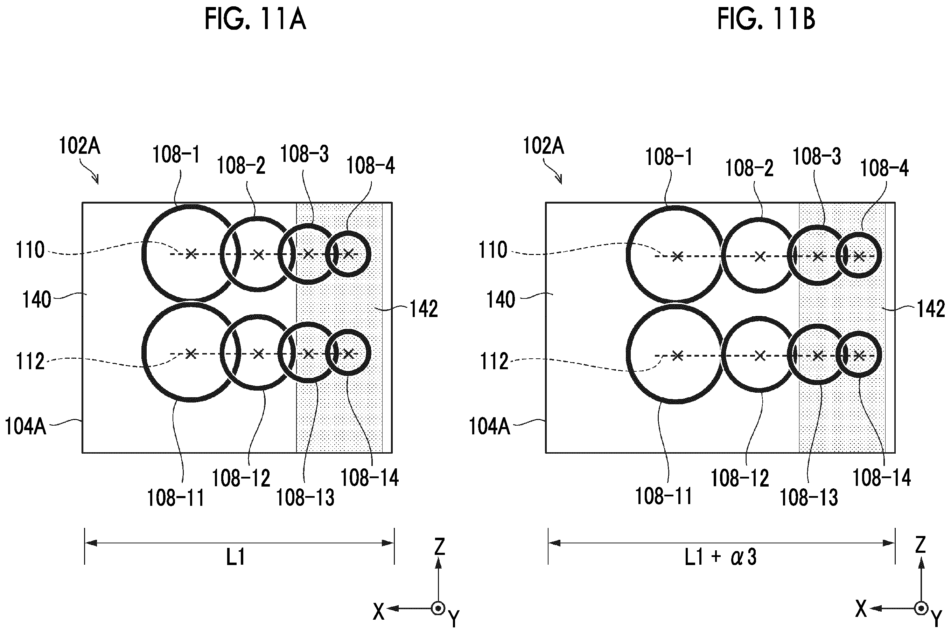

Figs. 11A and 11B are top perspective views of a first coil unit 102A. The first coil unit 102A comprises a first fixing belt 104A. The first fixing belt 104A comprises a stretch part 140 formed of a stretch material and a non-stretch part 142 formed of a non-stretch material.

The stretch part 140 is formed of a first stretch material that is stretchable in the X direction which is the elongation direction. The non-stretch part 142 is formed of a non-stretch material having a smaller elongation ratio in the X direction than the first stretch material. The elongation ratio of the non-stretch material is preferably less than 5%, and more preferably less than 1%.

Fig. 11A shows a non-elongated state of the first fixing belt 104A, and Fig. 11B shows an elongated state in which the first fixing belt 104A is elongated by α3 from the non-elongated state. In either state, the centers of the RF coils 108-1 to 108-4 are disposed on the straight line 110, and the centers of the RF coils 108-11 to 108-14 are disposed on the straight line 112. As shown in Fig. 10B, in the elongated state, the stretch part 140 is elongated, so that the distance between the RF coil 108-1 and the RF coil 108-2 and the distance between the RF coil 108-2 and the RF coil 108-3 increase. On the other hand, the non-stretch part 142 does not elongate, and the distance between the RF coil 108-3 and the RF coil 108-4 does not change.

As described above, the first fixing belt 104A can limit the part to be elongated, so that a part where performance of the RF coil 108 is stable can be constructed. A position and a length of the non-stretch part 142 in the X direction are not limited to the example shown in Figs. 11A and 11B and may be appropriately determined depending on a length of the first fixing belt 104A in the X direction, the size of the RF coil 108, and the like.

Here, an example in which a part of the first fixing belt 104A is formed of the non-stretch material has been described, but a part of the second fixing belt may be formed of the non-stretch material having a smaller elongation ratio than the second stretch material, or a part of the first fixing belt and a part of the second fixing belt may be formed of the non-stretch material.

In the present embodiment, the non-stretch material is provided at one location, but the present invention is not limited thereto. The non-stretch material may be provided at two or more locations in a belt shape in a direction perpendicular to a stretching direction. As a result, stretching can be finely controlled.

In addition, the first coil unit and the second coil unit may each comprise a stopper structure that restricts an elongation amount of the first fixing belt and the second fixing belt in the elongation direction from exceeding a certain amount.

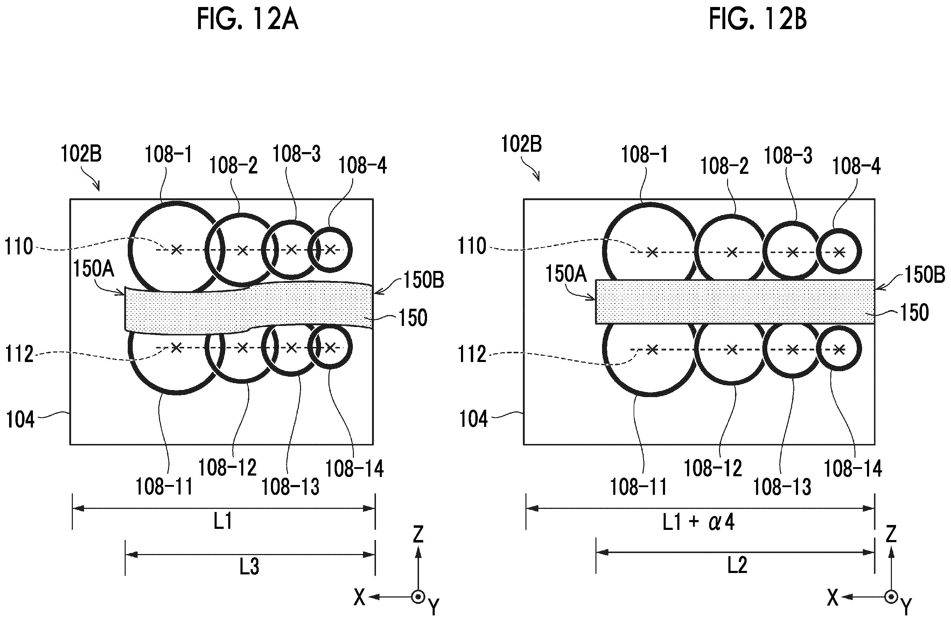

Figs. 12A and 12B are top perspective views of the first coil unit 102B. The first coil unit 102B comprises a stopper band 150 as the stopper structure. The stopper band 150 is formed of a non-stretch material having a smaller elongation ratio in the elongation direction than the first stretch material, and has a length L2 in the X direction. The length of the stopper band 150 in the X direction may be appropriately determined depending on the length of the first fixing belt 104 in the X direction, the size of the RF coil 108, and the like. A length of the stopper band 150 in the Z direction may be appropriately determined depending on strength of the stopper band 150 and the like. In the first fixing belt 104 in the non-elongated state, the stopper band 150 is fixed to the first fixing belt 104 such that an end part 150A and an end part 150B are spaced apart from each other by a predetermined distance L3 in the X direction, with a predetermined bend provided in advance. That is, L3 is smaller than L2.

Fig. 12A shows the non-elongated state of the first fixing belt 104, and Fig. 12B shows an elongated state in which the first fixing belt 104 is elongated by α4 from the non-elongated state. In the non-elongated state shown in Fig. 12A, the stopper band 150 is bent in the X direction. On the other hand, in the elongated state shown in Fig. 12B, the stopper band 150 is in a state with no bends, and the first fixing belt 104 does not further elongate in the X direction.

As shown in Fig. 12B, each of the plurality of RF coils 108 of the first coil unit 102B overlaps the adjacent RF coil 108. Therefore, the first fixing belt 104 does not elongate further to the point where the overlap between the RF coils 108 is lost.

As described above, with the first coil unit 102B comprising the stopper band 150, it is possible to reduce a region having no sensitivity to a relatively large subject, so that it is possible to obtain a relatively high sensitivity. A position where the stopper band 150 is disposed may be appropriately determined. In addition, it is preferable that a maximum elongation amount of the first fixing belt 104 restricted by the stopper band 150 is a maximum elongation amount at which the overlap between the adjacent RF coils can be maintained. In order to obtain appropriate performance of the RF coil array 100, it is preferable that the adjacent RF coils overlap each other by about 5% to 15%.

Here, an example in which the first coil unit 102B comprises the stopper band 150 as the stopper structure has been described, but the second coil unit may comprise the stopper structure, or the first coil unit and the second coil unit may each comprise the stopper structure.

### Fixing Structure of RF Coil Array

The first coil unit and the second coil unit may each have a structure that enables an end part of each of the first fixing belt and the second fixing belt on an opposite side to the distal end to be connected to the top plate, and may be connected to the top plate of the MRI apparatus.

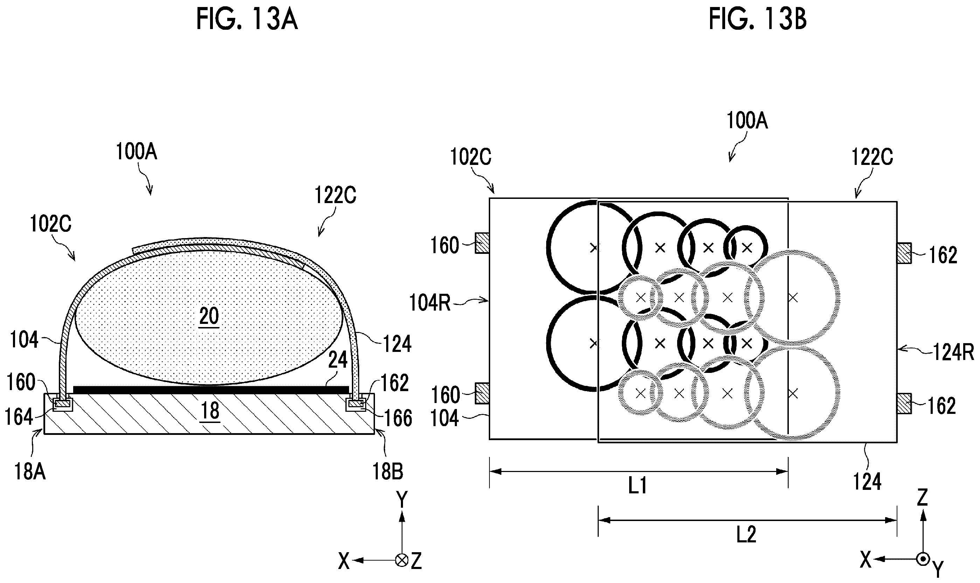

Figs. 13A and 13B are views showing an RF coil array 100A. Fig. 13A is a cross-sectional view similar to Fig. 6. Fig. 13B is a top perspective view showing the disposition of the RF coils when a state of Fig. 13A is viewed from the Z direction side, and is shown to be deployed in the X direction. In Fig. 13B, the first fixing belt 104 and the second fixing belt 124 are in a non-elongated state.

As shown in Figs. 13A and 13B, the RF coil array 100A comprises a first coil unit 102C and a second coil unit 122C. The first coil unit 102C comprises a plurality of rail hooks 160 at the proximal end 104R of the first fixing belt 104. The second coil unit 122C comprises a plurality of rail hooks 162 at the proximal end 124R of the second fixing belt 124.

In addition, the top plate 18 comprises a rail groove 164 along the Z direction on the first end 18A side and a rail groove 166 along the Z direction on the second end 18B side.

The first coil unit 102C is connected to the top plate 18 by engaging the plurality of rail hooks 160 with the rail groove 164. In addition, the second coil unit 122C is connected to the top plate 18 by engaging the plurality of rail hooks 162 with the rail groove 166.

As a result, the RF coil array 100A is closely mounted on the subject 20 and can fix the subject 20 to the top plate 18.

In addition, the plurality of rail hooks 160 are movable along the rail groove 164. In addition, the plurality of rail hooks 162 are movable along the rail groove 166.

As a result, the RF coil array 100A can be moved to a position in the Z direction according to the examination target region of the subject 20.

### Tightening Mechanism of RF Coil Array

The RF coil array may comprise a tightening mechanism for adjusting the elongation amount of at least one of the first fixing belt or the second fixing belt in the elongation direction after being mounted on the subject.

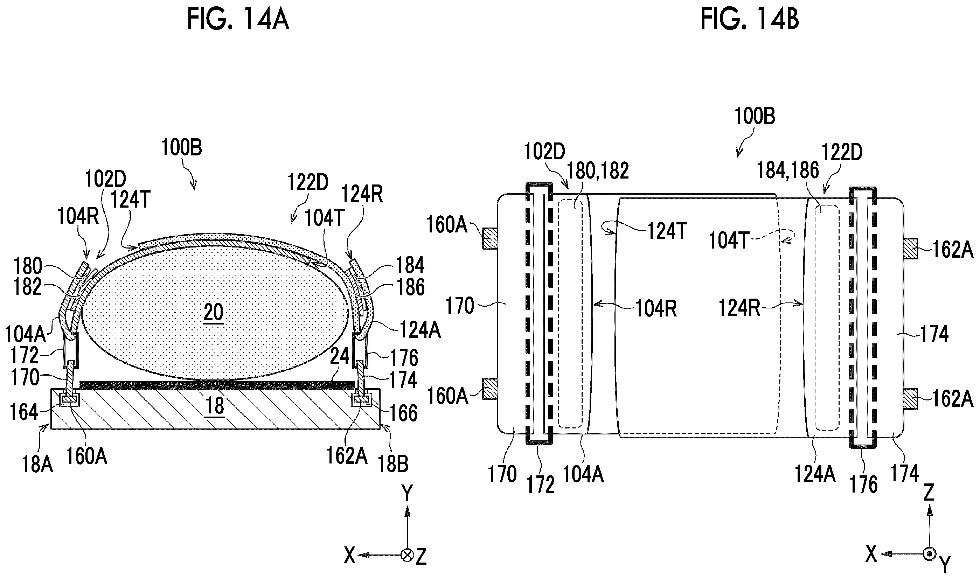

Figs. 14A and 14B are views showing an RF coil array 100B. Fig. 14A is a cross-sectional view similar to Fig. 6. Fig. 14B is a view of a state of Fig. 14A as viewed from the Z direction side, and is shown to be deployed in the X direction.

As shown in Figs. 14A and 14B, the RF coil array 100B comprises a first coil unit 102D and a second coil unit 122D. The first coil unit 102D comprises the first fixing belt 104A, a third fixing belt 170, and a rectangular ring 172 (an example of an "annular member") as the tightening mechanism.

In the first fixing belt 104A, a first surface 180 and a second surface 182 of a hook-and-loop fastener are provided at positions relatively close to the proximal end 104R on the same surface as the surface on which the first surface 106 and the second surface 126 (not shown in Figs. 14A and 14B, see Fig. 6) of the hook-and-loop fastener are disposed. For example, the first surface 180 is a loop surface, and the second surface 182 is a hook surface. The second surface 182 may be integrated with the first surface 106.

The first fixing belt 104A is connected to the rectangular ring 172 by passing the proximal end 104R through an opening of the rectangular ring 172, folding the proximal end 104R back, and coupling the first surface 180 and the second surface 182 of the hook-and-loop fastener with each other.

The third fixing belt 170 is passed through the opening (an example of "inside") of the rectangular ring 172 and is folded back, and both ends thereof are connected to each other. In addition, both ends of the third fixing belt 170 connected to each other comprise a plurality of rail hooks 160A.

The top plate 18 comprises a rail groove 164 on the first end 18A side along the Z direction. The first coil unit 102D is connected to the top plate 18 by engaging the plurality of rail hooks 160A with the rail groove 164.

The second coil unit 122D comprises a second fixing belt 124A, a fourth fixing belt 174, and a rectangular ring 176 as the tightening mechanism.

In the second fixing belt 124A, a first surface 184 and a second surface 186 of a hook-and-loop fastener are provided at positions relatively close to the proximal end 124R on a surface opposite to the surface on which the first surface 106 and the second surface 126 (not shown in Figs. 14A and 14B, see Fig. 6) of the hook-and-loop fastener are disposed. For example, the first surface 184 is a loop surface, and the second surface 186 is a hook surface.

The second fixing belt 124A is connected to the rectangular ring 176 by passing the proximal end 124R through an opening of the rectangular ring 176, folding the proximal end 124R back, and coupling the first surface 184 and the second surface 186 of the hook-and-loop fastener with each other.

The fourth fixing belt 174 is passed through the opening of the rectangular ring 176 and is folded back, and both ends thereof are connected to each other. In addition, both ends of the fourth fixing belt 174 connected to each other comprise a plurality of rail hooks 162A.

The top plate 18 comprises a rail groove 166 on the second end 18B side along the Z direction. The second coil unit 122D is connected to the top plate 18 by engaging the plurality of rail hooks 162A with the rail grooves 166.

In order to mount the RF coil array 100B on the subject 20, the user first connects the first coil unit 102D and the second coil unit 122D to the top plate 18.

Next, the user places the first coil unit 102 on the examination target region of the subject 20 placed on the top plate 18. The user further overlaps the second coil unit 122 on the first coil unit 102 mounted on the subject 20, and couples the first surface 106 of the hook-and-loop fastener of the first fixing belt 104 with the second surface 126 of the hook-and-loop fastener of the second fixing belt 124, and the first surface 106 of the hook-and-loop fastener of the second fixing belt 124 with the second surface 126 of the hook-and-loop fastener of the first fixing belt 104 (not shown in Figs. 14A and 14B, see Fig. 6). In a case where the size of the subject 20 is relatively large, the user brings the first fixing belt 104 and the second fixing belt 124 into the elongated state and then couples the first surfaces 106 and the second surfaces 126 of the hook-and-loop fasteners.

In this way, in the RF coil array 100 mounted on the subject 20, the first fixing belt 104A and the second fixing belt 124A can be tightened.

In order to tighten the first fixing belt 104A, the user uncouples the first surface 180 and the second surface 182 of the hook-and-loop fastener of the first fixing belt 104A, pulls the proximal end 104R of the first fixing belt 104A in a direction (for example, the Y direction) away from the rectangular ring 172, and couples the first surface 180 and the second surface 182 of the hook-and-loop fastener again.

In order to tighten the second fixing belt 124A, the user uncouples the first surface 184 and the second surface 186 of the hook-and-loop fastener of the second fixing belt 124A, pulls the proximal end 124R of the second fixing belt 124A in a direction away from the rectangular ring 176, and couples the first surface 184 and the second surface 186 of the hook-and-loop fastener again.

As a result, since the elongation of the RF coil array can be appropriately adjusted, stable image quality can be obtained.

Here, an example in which the first fixing belt 104A and the second fixing belt 124A can be tightened has been described, but only one of the first fixing belt 104A and the second fixing belt 124A may be tightened.

### Integrated Structure of First Coil Unit and Second Coil Unit

The first coil unit and the second coil unit may be connected to a connection unit to be integrated.

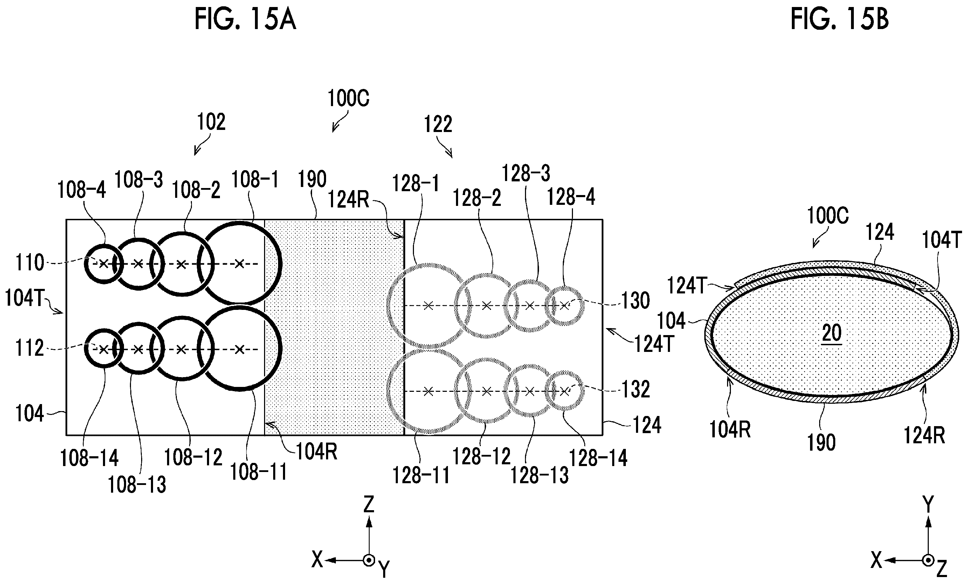

Figs. 15A and 15B are views showing an RF coil array 100C. Fig. 15A is a top perspective view of the RF coil array 100C, and Fig. 15B is a cross-sectional view similar to Fig. 6.

As shown in Figs. 15A and 15B, the RF coil array 100C comprises the first coil unit 102, the second coil unit 122, and a connection unit 190. The connection unit 190 is formed of a non-stretch material. The connection unit 190 has a rectangular shape, one end of which is connected to the proximal end 104R of the first fixing belt 104 and the other end of which is connected to the proximal end 124R of the second fixing belt 124. A length of the connection unit 190 in the X direction may be appropriately determined depending on the length of the first fixing belt 104 and the second fixing belt 124 in the X direction.

In order to mount the RF coil array 100C on the subject 20, the user wraps the RF coil array 100C around the examination target region of the subject 20 and couples the first surface 106 of the hook-and-loop fastener of the first fixing belt 104 with the second surface 126 of the hook-and-loop fastener of the second fixing belt 124, and the first surface 106 of the hook-and-loop fastener of the second fixing belt 124 with the second surface 126 of the hook-and-loop fastener of the first fixing belt 104 (not shown in Figs. 15A and 15B, see Fig. 6). In a case where the size of the subject 20 is relatively large, the user brings the first fixing belt 104 and the second fixing belt 124 into the elongated state and then couples the first surfaces 106 and the second surfaces 126 of the hook-and-loop fasteners.

As described above, the user can use the RF coil array 100C by wrapping the RF coil array 100C around the examination target region. The fixation of the subject 20 on which the RF coil array 100C is mounted to the top plate 18 may be performed using a separate member.

### RF Coil of Connection Unit

The connection unit may include a plurality of RF coils.

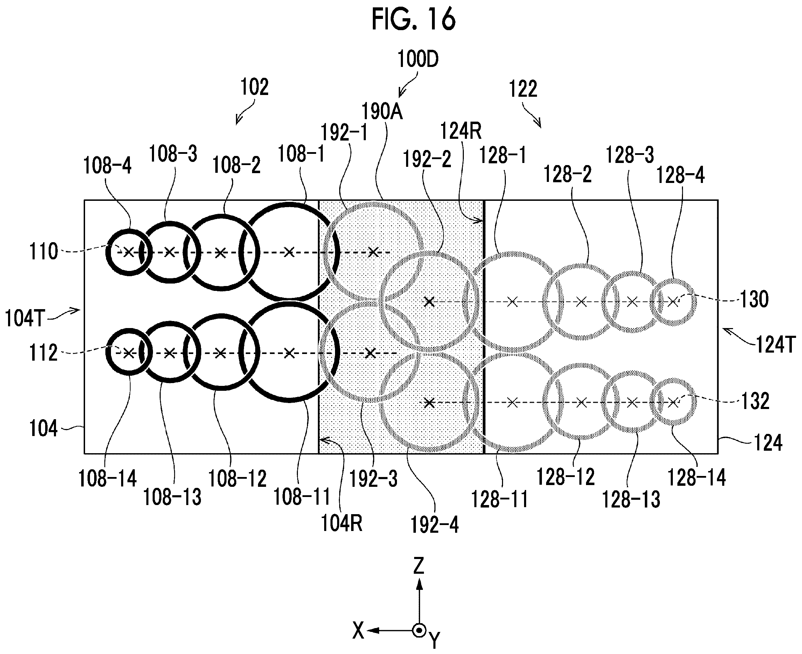

Fig. 16 is a view showing an RF coil array 100D. The RF coil array 100D comprises a connection unit 190A. The connection unit 190A includes RF coils 192-1, 192-2, 192-3, and 192-4. In a case where there is no need to distinguish between the RF coils 192-1 to 192-4, the RF coils 192-1 to 192-4 may be simply referred to as the RF coils 192.

The RF coil 192-1 has a center (indicated by a cross mark in Fig. 16) disposed on the straight line 110 parallel to the X direction. In addition, the RF coil 192-1 and the RF coil 108-1 partially overlap each other.

The RF coil 192-2 has a center disposed on the straight line 130 parallel to the X direction. In addition, the RF coil 192-2 and the RF coil 128-1 partially overlap each other.

The RF coil 192-3 has a center disposed on the straight line 112 parallel to the X direction. In addition, the RF coil 192-3 and the RF coil 108-11 partially overlap each other.

The RF coil 192-4 has a center disposed on the straight line 132 parallel to the X direction. In addition, the RF coil 192-4 and the RF coil 128-11 partially overlap each other.

The RF coil array 100D may output magnetic resonance signals received by the RF coils 192-1, 192-2, 192-3, and 192-4 through the cable or wireless communication via a signal processing circuit (not shown in Fig. 16) including a signal amplifier.

With the RF coil array 100D, the back coil 24 of the top plate 18 is not necessary. The number and disposition of the RF coils 192 of the connection unit 190A are not limited to the example shown in Fig. 16.

### Tightening Mechanism of RF Coil Array Having Integrated Structure

The RF coil array having the integrated structure may comprise a tightening mechanism for adjusting the elongation amount of at least one of the first fixing belt or the second fixing belt in the elongation direction after being mounted on the subject.

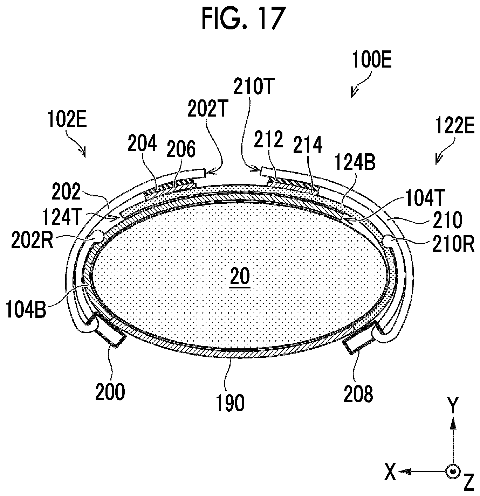

Fig. 17 is a view showing an RF coil array 100E and is a cross-sectional view similar to Fig. 6. As shown in Fig. 17, the RF coil array 100E comprises a first fixing belt 104B and a second fixing belt 124B. In addition, the RF coil array 100E comprises a rectangular ring 200, a first tightening belt 202, a rectangular ring 208, and a second tightening belt 210 as the tightening mechanism.

The rectangular ring 200 is fixed to a surface of the connection unit 190 on an opposite side to the surface that comes into contact with the subject 20. The rectangular ring 200 may be fixed to a surface of the first fixing belt 104B on an opposite side to the surface that comes into contact with the subject 20.

One end part 202R of the first tightening belt 202 is fixed to the surface of the first fixing belt 104B on the opposite side to the surface that comes into contact with the subject 20. A position where the end part 202R of the first tightening belt 202 is fixed is between the distal end 104T of the first fixing belt 104B and the rectangular ring 200.

The first tightening belt 202 is provided with a first surface 204 of a hook-and-loop fastener on the other end part 202T side. In addition, a second surface 206 of the hook-and-loop fastener is provided on a surface of the second fixing belt 124B on an opposite side to the surface that comes into contact with the subject 20.

The rectangular ring 208 is fixed to the surface of the connection unit 190 on the opposite side to the surface that comes into contact with the subject 20. The rectangular ring 208 may be fixed to the surface of the second fixing belt 124B on the opposite side to the surface that comes into contact with the subject 20.

One end part 210R of the second tightening belt 210 is fixed to the surface of the second fixing belt 124B on the opposite side to the surface that comes into contact with the subject 20. A position where the end part 210R of the second tightening belt 210 is fixed is between the distal end 124T of the second fixing belt 124B and the rectangular ring 208.

The second tightening belt 210 is provided with a first surface 212 of a hook-and-loop fastener on the other end part 210T side. In addition, a second surface 214 of the hook-and-loop fastener is provided on the surface of the second fixing belt 124B on the opposite side to the surface that comes into contact with the subject 20.

In order to mount the RF coil array 100E on the subject 20, the same method as in the case of the RF coil array 100C may be used in a state in which the first surface 204 and the second surface 206 of the hook-and-loop fastener are not coupled and the first surface 212 and the second surface 214 of the hook-and-loop fastener are not coupled.

Thereafter, in order to further tighten the first fixing belt 104B of the RF coil array 100E mounted on the subject 20, the user passes the first tightening belt 202 through an opening of the rectangular ring 200, folds the first tightening belt 202 back, and pulls the end part 202T of the first tightening belt 202 in a direction (for example, the Y direction) away from the rectangular ring 200 to couple the first surface 204 of the hook-and-loop fastener with the second surface 206.

In addition, in order to tighten the second fixing belt 124B, the user passes the second tightening belt 210 through an opening of the rectangular ring 208 and folds the second tightening belt 210 back, and pulls the end part 210T of the second tightening belt 210 in a direction away from the rectangular ring 208 to couple the first surface 212 of the hook-and-loop fastener with the second surface 214.

As a result, the RF coil array 100E is firmly fixed to the subject 20, and there is no deviation from the subject 20.

### Others

The technical scope of the present invention is not limited to the scope described in the above-described embodiments. The configurations and the like in each embodiment can be appropriately combined between each of the embodiments without departing from the gist of the present invention.

### Explanation of References

10: MRI apparatus
10A: MRI apparatus
12: magnet
14: examination space
16: bed
18: top plate
18A: first end
18B: second end
20: subject
22: fixing belt
24: back coil
26: upper abdominal coil
100: RF coil array
100A: RF coil array
100B: RF coil array
100C: RF coil array
100D: RF coil array
100E: RF coil array
102: first coil unit
102A: first coil unit
102B: first coil unit
102C: first coil unit
102D: first coil unit
104: first fixing belt
104A: first fixing belt
104B: first fixing belt
104R: proximal end
104T: distal end
106: first surface
108: RF coil
108-1: RF coil
108-2: RF coil
108-3: RF coil
108-4: RF coil
108-11: RF coil
108-12: RF coil
108-13: RF coil
108-14: RF coil
110: straight line
112: straight line
122: second coil unit
122C: second coil unit
122D: second coil unit
124: second fixing belt
124A: second fixing belt
124B: second fixing belt
124R: proximal end
124T: distal end
126: second surface
128: RF coil
128-1: RF coil
128-2: RF coil
128-3: RF coil
128-4: RF coil
128-11: RF coil
128-12: RF coil
128-13: RF coil
128-14: RF coil
130: straight line
132: straight line
140: stretch part
142: non-stretch part
150: stopper band
150A: end part
150B: end part
160: rail hook
160A: rail hook
162: rail hook
162A: rail hook
164: rail groove
166: rail groove
170: third fixing belt
172: rectangular ring
174: fourth fixing belt
176: rectangular ring
180: first surface
182: second surface
184: first surface
186: second surface
190: connection unit
190A: connection unit
192: RF coil
192-1: RF coil
192-2: RF coil
192-3: RF coil
192-4: RF coil
200: rectangular ring
202: belt
202R: end part
202T: end part
204: first surface
206: second surface
208: rectangular ring
210: belt
210R: end part
210T: end part
212: first surface
214: second surface

## Claims

1. An RF (radio frequency) coil array (100) for a magnetic resonance imaging apparatus (10), comprising:
a first coil unit (102) including a first fixing belt (104) formed of a first stretch material that is stretchable in a first direction and a plurality of RF coils (108) each independently disposed on the first fixing belt; and
a second coil unit (122) including a second fixing belt (124) formed of a second stretch material that is stretchable in the first direction and a plurality of RF coils (128) each independently disposed on the second fixing belt,
wherein each of the plurality of RF coils of the first coil unit has a center disposed on a first straight line, and the RF coils closer to a distal end of the first fixing belt have smaller diameters,
each of the plurality of RF coils of the second coil unit has a center disposed on a second straight line, and the RF coils closer to a distal end of the second fixing belt have smaller diameters,
the first straight line and the second straight line are disposed offset from each other in a second direction intersecting the first direction,
the first coil unit is mounted on a subject with the distal end (104T) of the first fixing belt directed from one side to the other side of the subject in the first direction, and
the second coil unit is mounted on the subject with the distal end (124T) of the second fixing belt directed from the other side to the one side of the subject in the first direction such that at least a part of the second coil unit overlaps the first coil unit.

2. The RF coil array according to claim 1,
wherein the first straight line and the second straight line are each parallel to the first direction.

3. The RF coil array according to claim 1 or 2,
wherein, in a case where the first coil unit (102) and the second coil unit (122) are mounted on the subject, any of the plurality of RF coils of the first coil unit and any of the plurality of RF coils of the second coil unit at least partially overlap each other.

4. The RF coil array according to claim 1 or 2,
wherein, in a case where the first coil unit (102) and the second coil unit (122) are mounted on the subject, the RF coil having a largest diameter among the plurality of RF coils of the first coil unit and the RF coil having a smallest diameter among the plurality of RF coils of the second coil unit at least partially overlap each other.

5. The RF coil array according to any one of claims 1 to 4,
wherein a part of at least one of the first fixing belt (104) or the second fixing belt (124) is formed of a non-stretch material having a smaller elongation ratio in the first direction than the first stretch material and the second stretch material.

6. The RF coil array according to any one of claims 1 to 5, further comprising:
a stopper structure that restricts elongation of the first fixing belt and the second fixing belt in the first direction beyond a predetermined amount,
preferably, wherein the stopper structure includes a stopper band (150) that is formed of a non-stretch material having a smaller elongation ratio in the first direction than the first stretch material and the second stretch material and that is provided on the first fixing belt and the second fixing belt with a bend provided in advance in the first direction.

7. The RF coil array according to any one of claims 1 to 6,
wherein, in the first coil unit (102) and the second coil unit (122), an end part of each of the first fixing belt and the second fixing belt on an opposite side to the distal end is connected to a top plate on which the subject is placed.

8. The RF coil array according to claim 7,
wherein the first fixing belt (104) and the second fixing belt (124) each comprises a rail hook that is engaged with a rail groove provided in the top plate and that is movable along the rail groove at the end part on the opposite side.

9. The RF coil array according to claim 7 or 8, further comprising:
a tightening mechanism for adjusting an elongation amount of at least one of the first fixing belt or the second fixing belt in the first direction after being mounted on the subject,
preferably,
wherein the tightening mechanism comprises an annular member (172) connected to the top plate, and
the end part of at least one of the first fixing belt or the second fixing belt on the opposite side to the distal end is passed through an inside of the annular member and folded back to be fixed.

10. The RF coil array according to any one of claims 1 to 6,
wherein the first coil unit (102) and the second coil unit (122) are each connected to a connection unit to be integrated.

11. The RF coil array according to claim 10,
wherein the connection unit includes a plurality of RF coils.

12. The RF coil array according to claim 10 or 11, further comprising:
a tightening mechanism for adjusting an elongation amount of at least one of the first fixing belt or the second fixing belt in the first direction after being mounted on the subject,
preferably,
wherein the tightening mechanism comprises a tightening belt connected to at least one of the first fixing belt or the second fixing belt, and an annular member (172), and
the tightening belt is passed through an inside of the annular member and folded back to be fixed.

13. The RF coil array according to any one of claims 1 to 12,
wherein each of the plurality of RF coils of the first coil unit overlaps an adjacent RF coil, and
each of the plurality of RF coils of the second coil unit overlaps an adjacent RF coil.

14. The RF coil array according to any one of claims 1 to 13,
wherein the first fixing belt (104) and the second fixing belt (124) comprise first surfaces and second surfaces of a pair of hook-and-loop fasteners each having the first surface and the second surface engaging with each other, at positions where the first surface and the second surface come into contact with each other in a case where the first coil unit and the second coil unit overlap the subject and are mounted on the subject.

15. The RF coil array according to any one of claims 1 to 14,
wherein the first coil unit is mounted on the subject with the first fixing belt elongated, and
the second coil unit is mounted on the subject with the second fixing belt elongated.
